(19) Europäisches Patentamt
European Patent Office
Office européen des brevets

(11) Numéro de publication: **0 113 330**
**B1**

(12) # FASCICULE DE BREVET EUROPEEN

(45) Date de publication du fascicule du brevet: **01.03.89**

(51) Int. Cl.⁴: **C 07 C 103/84,** A 61 K 31/195 // (A61K31/195,31:675), (A61K31/195, 31:70)

(21) Numéro de dépôt: **83870139.9**

(22) Date de dépôt: **20.12.83**

(54) **Composés énamides acylés, compositions pharmaceutiques les contenant, utilisation de ces composés et procédé de préparation de ces composés.**

**Jointe à la demande no. 84900016.1/0161249 (numéro de dépôt/numéro de publication de la demande européenne) par décision du 14.07.86.**

(30) Priorité: **27.12.82 FR 8221965**

(43) Date de publication de la demande: **11.07.84 Bulletin 84/28**

(45) Mention de la délivrance du brevet: **01.03.89 Bulletin 89/9**

(84) Etats contractants désignés: **AT BE CH DE FR GB IT LI LU NL SE**

(56) Documents cités:
**DE-A- 2 201 478**
**US-A- 2 449 191**

**SYNTHESIS, no. 7, juillet 1977 A.J. KOLAR et al.: "A convenient, Large-Scale Preparation of 2-Acetamidoacrylic Acid and its Methyl Ester", pages 457-459.**

(73) Titulaire: **BIOTEC (Société Anonyme), Avenue Louise, 479 (bte 47), B-1050 Bruxelles (BE)**

(72) Inventeur: **Viehe, Heinz, Clos du Paradis, 7 Grand Manil, B-1350 Limal (BE)**
Inventeur: **Hervens, Françoise, Leuvensebaan, 354, B-3051 Sint-Agatha-Rode (BE)**
Inventeur: **Roberfroid, Marcel, 6, Place des Giroflées, B-1348 Louvain-La-Neuve (BE)**

(74) Mandataire: **Van Malderen, Michel et al, p.a. Freylinger & Associés 22 avenue J.S. Bach (bte 43), B-1080 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne des composés énamides acylés constitués par des énamides acylés ainsi que par leurs sels, leurs complexes et leurs dérivés acylés et alkylés. L'invention concerne également des compositions pharmaceutiques les contenant, utilisables en particulier pour la prévention ou le traitement des cancers, l'utilisation de ces composés pour la prévention des cancers ainsi qu'un procédé pour la préparation de ces composés.

Les composés énamides acylés selon l'invention sont des composés répondant à la formule générale:

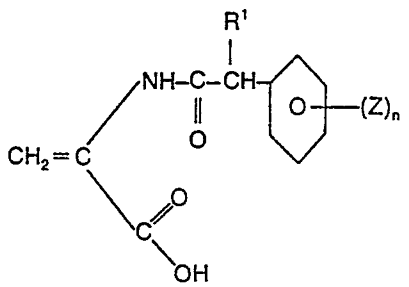

(I)

dans laquelle

– Z représente un groupement donneur d'électrons,

– n vaut 1, 2 ou 3, et

– $R^1$ représente l'hydrogène, un halogène, un groupement aliphatique, substitué ou non, et comptant de 1 à 4 atomes de carbone ou un groupement aromatique comptant de 5 à 8 atomes de carbone,

ou des sels, complexes, dérivés alkylés ou dérivés acylés de ces composés.

Les sels de ces composés peuvent être par exemple des sels d'ammonium, de métaux alcalins tels que le sodium ou le potassium, de métaux alcalino-terreux tels que le magnésium et le calcium et de bases organiques. Les complexes de ces composés peuvent être ceux formés par exemple avec des sels métalliques quelconques. Par dérivés acylés de ces composés, on entend des dérivés tels que les esters et les amides de ces composés; de préférence le noyau aromatique porteur de Z ne contient pas de substituants acylés.

Habituellement, le groupement donneur est un halogène ou un groupement sélectionné parmi les groupements $OR^2$, $SR^2$, $SeR^2$, $N=R^2$ ou $NR^2R^3$ dans lesquels l'oxygène, le soufre, le sélénium et l'azote peuvent faire partie d'un cycle et $R^2$ et $R^3$ peuvent être identiques ou différents et représentent l'hydrogène ou un groupement alkyle, substitué ou non comptant de 1 à 6 atomes de carbone. De préférence, le groupement donneur est un groupement $OR^2$ où $R^2$ représente l'hydrogène ou un groupement alkyle substitué ou non comptant de 1 à 6 atomes de carbone. De manière particulièrement préférée, le groupement donneur est un groupement méthoxy $-OCH_3$.

Habituellement, $R^1$ représente l'hydrogène, un halogène, ou un groupement organique quelconque. De préférence, $R^1$ représente l'hydrogène, ou un groupement aliphatique, substitué ou non, et comptant de 1 à 4 atomes de carbone, ou un groupement aromatique comptant de 5 à 8 atomes de carbone. De manière particulièrement préférée, $R^1$ représente l'hydrogène.

Les composés énamides acylés préférés selon l'invention sont les (méthoxyphénylacétyl)déhydroalanines de formule

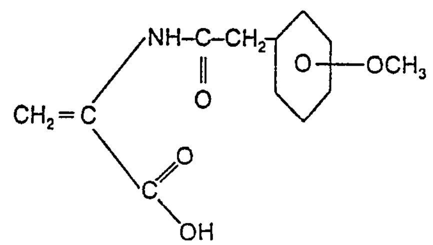

(II)

Parmi ceux-ci, le composé énamide acylé préféré est celui dans lequel le substituant méthoxy se trouve en position para.

Des dérivés α-acylamino d'acrylate d'alkoyle ayant des propriétés insecticides ont été décrits dans le document DE-A-2 201 478 (BEECHAM).

Dans le document US-A-2 449 191 (O.K. BEHRENS), on a décrit des α-aminoacides N-substitués par un reste phénylacétyle. Ces composés sont incorporés dans des milieux de culture pour accroître la production de pénicilline.

Le document SYNTHESIS, N° 7, juillet 1977 A.J. KOLAR et al.: «A convenient, large-scale preparation of 2-acetamidoacrylic acid and its methyl ester», Pages 457–458 décrit, sans indication d'usage, un procédé de production de l'acide 2-acétamidoacrylique et de son ester méthylique.

Aucun de ces documents ne mentionne une utilisation thérapeutique des composés selon la présente invention.

Un procédé pour la préparation de composés énamides acylés selon l'invention consiste à faire réagir avec l'acide pyruvique un amide de formule

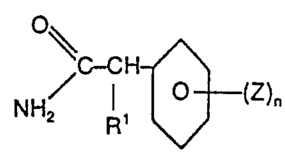

où $R^1$, Z et n sont tels que définis ci-avant. La réaction peut être réalisée en mettant 1 mole d'amide en présence de 1,2 mole d'acide pyruvique fraîchement distillé en les portant à reflux dans le benzène dans un appareil muni d'un séparateur d'eau. Le reflux est arrêté quand la séparation d'eau cesse. On attend la formation d'un précipité, on filtre et traite le précipité avec une solution saturée en $NaHCO_3$. La fraction insoluble en milieu basique, qui est constituée d'amide n'ayant pas réagi est séparée et on ajoute alors progressivement au filtrat de l'acide chlorhydrique concentré jusqu'à l'obtention d'un pH voisin de 3,5 et d'un précipité. Après filtration et recristallisation dans l'éthanol, on obtient des rendements situés entre 30% et 95%. Il peut arriver que l'on n'obtienne pas de précipité après le reflux en

solution benzénique; dans ce cas, on évapore le benzène et on traite le résidu de la même manière qu'indiqué ci-dessus.

Les composés énamides acylés selon l'invention peuvent également être préparés par toute autre synthèse organique appropriée. On peut par exemple appliquer le procédé décrit dans le brevet FR-B-2 349 567 (SUMITOMO CHEMICAL COMPANY).

Les composés énamides acylés selon l'invention peuvent être utilisés comme médicaments, et notamment pour le traitement, en médecine humaine ou en médecine vétérinaire, de toutes les proliférations tumorales malignes telles que les cancers d'origines diverses et ce quel que soit l'âge et le type histologique du tissu aux dépens duquel elles se sont formées. Ils peuvent également être utilisés pour la prévention de ces mêmes maladies, par exemple sous forme d'additif alimentaire.

L'invention concerne également les compositions pharmaceutiques contenant comme ingrédient actif un composé énamide acylé tel que défini ci-avant. Ces compositions peuvent contenir en outre une substance antinéoplasique pour une utilisation simultanée, séparée ou étalée dans le temps en thérapie cytostatique. Les substances antinéoplasiques connues présentent généralement le grave inconvénient d'avoir un degré de toxicité élevé lequel provoque des effets secondaires particulièrement néfastes tels que des dommages aux tissus normaux. Les compositions pharmaceutiques selon l'invention présentent à la fois un degré de toxicité réduit et un effet thérapeutique amélioré par rapport aux compositions connues contenant seulement des substances antinéoplasiques.

Par substance antinéoplasique, on entend désigner toute substance utilisable dans le but de détruire les cellules cancéreuses où d'en empêcher l'apparition et la prolifération. On connaît comme substances antinéoplasiques, des composés de natures chimiques très variables parmi lesquelles figurent des agents alkylants, comme le cyclophosphamide, le melphalan, le chlorambucil, la chlormétine et d'autres moutardes à l'azote, les éthylène-imines parmi lesquelles le thiothépa, les éthers sulfoniques parmi lesquels le busulfan, des antimétabolites comme le méthotrexate et d'autres antifoliques, la mercaptopurine et autres antipuriques, le fluorouracile, la cytarabine et autres antipyrimidiques, l'azasérine, des modificateurs hormonaux comme les androgènes, les œstrogènes, les progestatifs et les glucocorticoïdes, des enzymes comme l'asparaginase, des antibiotiques parmi lesquels la bléomycine, la dactinomycine, la daunorubicine, la méractinomycine, la mitomycine, le rufocromycine, la méthramycine, la doxorubicine (parfois appelée l'adriamycine), des extraits de plantes comme les alcaloïdes de la pervenche et de la colchique et les autres poisons fusoriaux, des isotopes radioactifs comme le radiophosphate de sodium, de radioiodure de sodium ou d'autres composés tels le méthylglyoxalbis (guanylhydrazone), les dérivés de la podophyllotoxine, le mitotane, le pipobromane, les nitroso-urées et l'hydroxy-urée. Les substances antinéoplasiques présentes de préférence dans les compositions pharmaceutiques selon l'invention sont les moutardes à l'azote, les antibiotiques et les antipyrimidiques et tout particulièrement le cyclophosphamide, la daunorubicine, la doxorubicine et le fluorouracile.

Les compositions pharmaceutiques selon l'invention peuvent être utilisées pour le traitement et la prévention, en médecine humaine ou en médecine vétérinaire, de toutes les proliférations tumorales malignes telles que les cancers.

Les compositions pharmaceutiques selon l'invention peuvent aussi contenir d'autres substances actives utilisables dans les compositions pharmaceutiques pour la prévention ou le traitement des cancers, et notamment de l'acide ascorbique.

Habituellement, elles contiennent également des additifs de formulation permettant de les administrer commodément, par exemple sous forme de poudres, tablettes, pommades, lotions, capsules, dragées, gélules, ampoules, sirops, émulsions, suppositoires, injections ou solutions. Ces additifs peuvent être des supports ou des agents auxiliaires pharmaceutiques, solides ou liquides, organiques ou inorganiques, appropriés tels que l'eau, les solvants organiques de type paraffinique, la gélatine, le lactose, l'amidon, le stéarate de magnésium, le talc, les graisses et huiles végétales et animales, la gomme, les polyalkylèneglycols ou des liants et autres agents habituels.

Les compositions pharmaceutiques selon l'invention contiennent en général entre 10% et 99% en poids de composés énamides acylés selon l'invention et éventuellement de substance antinéoplasique. Le rapport pondéral entre les quantités de substance antinéoplasique et de composés énamides acylés selon l'invention présentes dans les compositions selon l'invention est compris en général entre 1/10 et 10/1, et de préférence entre 1/4 et 4/1.

Les composés énamides acylés et les compositions pharmaceutiques selon l'invention peuvent être administrés pour traiter ou prévenir des proliférations tumorales malignes telles que les cancers à tous les mammifères mais en particulier à des patients humains sous forme d'une ou plusieurs «unités de dosage» en quantité pharmaceutiquement efficace.

Tous les modes d'administration peuvent convenir, tels que l'administration par voie orale, rectale ou parentérale ainsi que par application épidermique du type onguents divers ou pastilles épidermiques. Par mode d'administration parentéral on entend les injections intraveineuses, intramusculaires ainsi que la perfusion.

Les compositions pharmaceutiques selon l'invention peuvent être administrées en médecine humaine par voie orale en unités de dosage comprenant en général au moins 0,05 mg et jusqu'à 500 mg, de préférence de 0,5 mg à 50 mg, de composés énamides acylés selon l'invention et éventuellement de substance antinéoplasique, avec un

support non toxique acceptable en pharmacie. On peut éventuellement administrer les compositions pharmaceutiques selon l'invention et les substances antinéoplasiques séparément et à des moments différents; on préfère dans ce cas administrer les compositions pharmaceutiques selon l'invention avant d'administrer les substances antinéoplasiques et ce dans un délai s'étendant entre 1 h et 24 h.

Par «unité de dosage», on entend une dose unitaire qui peut être administrée à un patient et peut facilement être manipulée et conditionnée, en restant sous forme d'une dose unitaire physiquement stable, comprenant l'ingrédient actif soit seul, soit en mélange avec des diluants ou supports pharmaceutiques solides ou liquides.

Sous la forme d'unités de dosage, les compositions pharmaceutiques selon l'invention peuvent être administrées une à plusieurs fois par jour, à intervalles appropriés, mais toujours selon l'état du patient et en fonction des prescriptions du médecin. La dose journalière appropriée des compositions selon l'invention varie en général de 0,01 mg à 50 mg par kg de poids corporel.

Pour le mode d'administration parentéral, notamment les injections intraveineuses ou intramusculaires, les compositions selon l'invention sont administrées, par exemple en solutions ou suspensions aqueuses, sous forme d'une unité de dosage contenant de 0,5 mg à 50 mg de composé énamide acylé selon l'invention et éventuellement de substance antinéoplasique, à dissoudre ou suspendre immédiatement avant l'utilisation ou prêt à l'emploi avec un véhicule acceptable en pharmacie par exemple une ampoule injectable.

Dans une thérapie faisant appel à un traitement continu, les gélules ou capsules peuvent être la forme appropriée de préparation pharmaceutique en raison des effets de longue durée obtenus lorsque le médicament est administré par voie orale.

Les exemples suivants illustrent l'invention.

Exemple 1

Préparation de para(méthoxyphénylacétyl)déhydroalanine

Dans un ballon en verre de deux cent cinquante millilitres muni d'un séparateur d'eau, on introduit à température ambiante 4,95 g ($3\cdot10^{-2}$ mole) de para(méthoxyphényl)acétamide. On ajoute ensuite 3,2 g ($3,6\cdot10^{-2}$ mole) d'acide pyruvique fraîchement distillé et 120 millilitres de benzène. On porte alors ce mélange à reflux; au début du reflux, la solution devient limpide puis se trouble progressivement par suite de la précipitation de la para(méthoxyphénylacétyl)déhydroalanine. On continue l'opération de reflux jusqu'à la fin de la séparation d'eau ce qui nécessite environ quarante-huit heures puis on chasse le benzène sous vide.

On extrait la para(méthoxyphénylacétyl)déhydroalanine au moyen d'une solution saturée de $NaHCO_3$ et on sépare par filtration 1,7 g de para(méthoxyphényl)acétamide qui n'a pas réagi.

Une fois la filtration terminée, on ajoute goutte à goutte quelques millilitres d'acide chlorhydrique jusqu'à obtention d'un pH voisin de 3 ou 4. La para(méthoxyphénylacétyl)déhydroalanine précipite et est récupérée par filtration et recristallisation dans l'éthanol. On obtient 4,45 g de para(méthoxyphénylacétyl)déhydroalanine avec un rendement de 62%. Le point de fusion du produit obtenu est mesuré à 186 °C. Le spectre infrarouge (KBr) révèle des bandes d'absorption à 3400 $cm^{-1}$ (-OH), 1700 $cm^{-1}$ (C=O), 1620 $cm^{-1}$ (C=C), 1510 $cm^{-1}$, 1420 $cm^{-1}$ et 1300 $cm^{-1}$. La spectrographie de masse révèle un pic à 235.

Le spectre de résonance magnétique nucléaire de la para(méthoxyphénylacétyl)déhydroalanine dissoute dans le diméthylsulfoxyde fournit les déplacements chimiques suivants en valeurs de δ (ppm):

| | | |
|---|---|---|
| 1 H | s | 8,96 |
| 2 H | m | 7,20 |
| 2 H | m | 6,86 |
| 1 H | s | 6,32 |
| 1 H | s | 5,69 |
| 3 H | s | 3,73 |
| 2 H | s | 3,61 |

Exemple 2

Composition pharmaceutique

On prépare une composition pharmaceutique en mélangeant 4 g de para(méthoxyphénylacétyl)déhydroalanine obtenu selon l'exemple 1 et 3,2 g de cyclophosphamide.

On injecte à un groupe de 10 souris pesant chacune environ 30 g, un million de cellules cancéreuses «Taper Liver Tumor» par voie intrapéritoniale; 48 heures après cette injection, on administre, également par voie intrapéritoniale, aux souris la composition pharmaceutique décrite ci-dessus à raison de 180 mg par kg de souris.

A titre comparatif, un groupe de dix souris semblables, auxquelles on a également administré un million de cellules cancéreuses «Taper Liver Tumor», est soumis uniquement à l'administration de cyclophosphamide par injection intrapéritoniale quarante huit heures après l'administration des cellules cancéreuses et un groupe de dix autres souris semblables, auxquelles on a également administré un million de cellules cancéreuses «Taper Liver Tumor», n'est soumis à aucun traitement ultérieur.

Parmi le groupe de souris n'ayant subi aucun traitement ultérieur, toutes meurent entre le dix-huitième et le vingt-troisième jour.

Parmi le groupe de souris ayant été uniquement traitées par du cyclophosphamide, toutes meurent entre le vingt-deuxième jour et le vingt-cinquième jour.

Parmi le groupe de souris ayant été traitées par la composition pharmaceutique selon l'invention, les souris ne meurent qu'entre le vingt-huitième et le trente-neuvième jour.

Le traitement de l'ascite par la composition pharmaceutique selon l'invention a donc permis de prolonger sensiblement la durée de vie des souris atteintes.

Exemple 3
Compositions pharmaceutiques contenant du cyclophosphamide et de la para(méthoxyphénylacétyl)déhydroalanine – Variation de la quantité de para(méthoxyphénylacétyl)déhydroalanine introduite

On injecte à des groupes de douze souris mâles, adultes de souche NMRI d'un poids corporel d'environ trente grammes, un million de cellules cancéreuses «Taper Liver Tumor» par voie intrapéritoniale; 48 heures après cette injection, on administre, également par voie intrapéritoniale, aux souris diverses compositions pharmaceutiques selon l'invention en faisant varier la quantité de para(méthoxyphénylacétyl)déhydroalanine présente dans la composition administrée, ou des compositions ne contenant que du cyclophosphamide.

On mesure ensuite trois paramètres

(a) la variation du poids moyen des souris au cinquième jour après inoculation de la tumeur cancéreuse (V.P.M.)
(b) la médiane de survie de 12 souris traitées par une composition pharmaceutique selon l'invention ou traitées par le cyclophosphamide seul (M.S.)
(c) la prolongation de la survie des souris traitées par une composition selon l'invention par rapport aux souris-témoin traitées par le cyclophosphamide seul (P.S.).

Les résultats obtenus sont regroupés dans le tableau ci-après, la para(méthoxyphénylacétyl)déhydroalanine étant désignée sous la dénomination S 86. Ils mettent en évidence une prolongation de la survie des souris lorsque l'on administre une composition pharmaceutique comprenant des doses de 25,50 ou 100 mg/kg de S 86 dont la dose léthale ($LD_{50}$) est supérieure à 400 mg par kg de poids corporel sur la souris, cette valeur étant le maximum injectable sur la souris, par suite de la faible solubilité du produit.

Tableau I

| Composition pharmaceutique et dose administrée | | V.P.M. | M.S. (jour) | P.S. (%) |
|---|---|---|---|---|
| Cyclophosphamide | 80 mg/kg | −1 | 24 | |
| Cyclophosphamide<br>+ S 86 | 80 mg/kg<br>400 mg/kg | −7,8 | 18 | −25 |
| Cyclophosphamide<br>+ S 86 | 80 mg/kg<br>200 mg/kg | −5 | 24 | 0 |
| Cyclophosphamide<br>+ S 86 | 80 mg/kg<br>100 mg/kg | −3 | 26 | +15 |
| Cyclophosphamide<br>+ S 86 | 80 mg/kg<br>50 mg/kg | −2 | 27,5 | +15 |
| Cyclophosphamide<br>+ S 86 | 80 mg/kg<br>25 mg/kg | −4 | 26 | + 9 |

Exemple 4
Compositions pharmaceutiques contenant du cyclophosphamide et de la para(méthoxyphénylacétyl)déhydroalanine – Variation de la quantité de cyclophosphamide introduite

On injecte à des groupes de douze souris mâles, adultes de souche NMRI d'un poids corporel d'environ 30 g, un million de cellules cancéreuses «Taper Liver Tumor» par voie intrapéritoniale; 48 heures après cette injection, on administre, également par voie intrapéritoniale, aux souris diverses compositions pharmaceutiques selon l'invention en faisant varier la quantité de cyclophosphamide présente dans la composition administrée, ou des compositions ne contenant que du cyclophosphamide.

On mesure ensuite trois paramètres:

(a) la variation du poids moyen des souris au cinquième jour après inoculation de la tumeur cancéreuse (V.P.M.),
(b) la médiane de survie (M.S.) de 12 souris traitées par une composition pharmaceutique selon l'invention ou traitées par le cyclophosphamide seul,
(c) la prolongation de la survie (P.S.) des souris traitées par une composition pharmaceutique selon l'invention par rapport aux souris témoin traitées avec la même dose de cyclophosphamide.

Les résultats obtenus sont regroupés dans le tableau ci-après, la para(méthoxyphénylacétyl)déhydroalanine étant désignée sous la dénomination S. 86. Ils montrent une nette prolongation du temps de survie lors de l'administration de compositions pharmaceutiques selon l'invention comprenant des doses de cyclophosphamide égales ou supérieures à 360 mg/kg. On remarque également que le poids moyen des souris varie plus faiblement pour les souris traitées par des compositions pharmaceutiques selon l'invention que pour les souris traitées par le cyclophosphamide.

Tableau II

| Composition pharmaceutique et dose administrée | | V.P.M. | M.S. (jour) | P.S. (%) |
|---|---|---|---|---|
| Cyclophosphamide | 80 mg/kg | − 2,2 | 21 | |
| Cyclophosphamide<br>+ S 86 | 80 mg/kg<br>50 mg/kg | − 2,6 | 22 | + 5 |
| Cyclophosphamide | 180 mg/kg | − 4,4 | 23 | |
| Cyclophosphamide<br>+ S 86 | 180 mg/kg<br>50 mg/kg | − 3 | 18,5 | − 17 |
| Cyclophosphamide | 360 mg/kg | − 6,6 | 31 | |
| Cyclophosphamide<br>+ S 86 | 360 mg/kg<br>50 mg/kg | − 10 | 34 | + 10 |
| Cyclophosphamide | 450 mg/kg | − 14 | 20 | |
| Cyclophosphamide<br>+ S 86 | 450 mg/kg<br>50 mg/kg | − 10,4 | 34 | + 70 |
| Cyclophosphamide | 540 mg/kg | − 18,6 | 12 | |
| Cyclophosphamide<br>+ S 86 | 540 mg/kg<br>50 mg/kg | − 11,6 | 38,5 | + 204 |

Exemple 5

Inhibition de la toxicité de la doxorubicine in vivo

L'injection sous-cutanée d'une dose unique de doxorubicine (10 mg/kg) à des rats entraîne, au niveau hépatique, une perte de 20% du contenu minimal en cytochrome P450, une chute de l'activité benzo(a)pyrene hydroxylase et une diminution de 85% de l'action aldrine monoxygènase. L'administration de cystéamine utilisée comme substance antitoxique de référence ne protège pas contre l'effet de la doxorubicine au niveau du cytochrome P450. Par contre la chute de l'activité benzo(a)pyrène hydroxylase n'est plus que de 20% et la diminution de l'activité aldrine monooxygènase est réduite à 40%.

On a injecté à des rats une dose unique (10 mg/kg) d'une composition contenant des quantités pondérales égales de doxorubicine et de para(méthoxyphénylacétyl)déhydroalanine. On a alors observé une augmentation de 60% du contenu minimal en cytochrome P450, une augmentation de 20% de l'activité benzo(a)pyrènehydroxylase et une diminution de 5% de l'action aldrine monooxygènase.

De ces essais, il ressort que le composé énamide acylé selon l'invention protège complètement contre les trois effets toxiques précités de la doxorubicine.

Exemple 6

Composition pharmaceutique

On prépare une composition pharmaceutique en mélangeant 4 g de para(méthoxyphénylacétyl)déhydroalanine obtenu selon l'exemple 1 et 0,2 g de doxorubicine.

On injecte à un groupe de six rats de souche LOU/2 et de poids corporel moyen égal à 160 g un million de cellules de leucémie L311 par voie intrapéritoniale. La leucémie L311 est une tumeur transplantable chez les rats de souche LOU/2 décrite dans l'article C. Deckers, F. Mare, L. Deckers-Passeu et A. Trouet, Adriamycin Review, Part I, 1975, pages 79 à 86.

On administre, 48 heures après cette injection, également par voie intrapéritoniale, aux rats la composition pharmaceutique décrite ci-dessus à raison de 105 mg par kg de rat.

A titre comparatif, un groupe de douze rats semblables, auxquels on a également administré un million de cellules de leucémie R311, est soumis uniquement à l'administration de doxorubicine par voie intrapéritoniale 48 heures après l'administration des cellules de leucémie R311, et un groupe de douze rats semblables, auxquels on a également administré un million de cellules de leucémie R311, n'est soumis à aucun traitement ultérieur.

La longueur de la survie moyenne après administration des cellules de leucémie est de 15,9 jours pour les rats leucémiques non traités, de 27,6 jours pour ceux traités uniquement à la doxorubicine et de 33,6 jours pour les rats traités à l'aide de la composition selon l'invention.

Pour les rats leucémiques non traités, le premier décès survient quatorze jours après l'administration des cellules de leucémie. Le délai est de seize jours pour les rats traités à la doxorubicine et de vingt et un jours pour les rats traités à l'aide de la composition selon l'invention.

Le traitement de la leucémie par les compositions selon l'invention a donc permis de prolonger sensiblement la durée de vie des rats leucémiques.

Exemples 7 à 11

De manière analogue au mode opératoire décrit dans l'exemple 1, on a préparé les composés de la formule générale (I) dans laquelle n = 1 et Z a la signification indiquée.

| Exemple n° | Z | Rendement (%) | Produit de fusion (°C) |
|---|---|---|---|
| 7 | $- OC_2H_5$ | 41 | 142 |
| 8 | $- OCH_2-C_6H_5$ | 62 | 188 |
| 9 | $- OCH_2-CH_2-CH_3$ | 30 | 178 |
| 10 | $- OCH(CH_3)_2$ | 18 | 173 |
| 11 | $- SCH_3$ | 71 | 209 |

Ces composés présentent un effet détoxifiant similaire à celui du composé de l'Exemple 1.

Exemple 12
Préparation de α (paraméthoxyphényl)acétamido acrylate de sodium(N-(paraméthoxyphénylacétyl)dehydro alaninate de sodium)

On dissout 2,35 g ($10^{-2}$ mole) d'acide (para méthoxyphénylacétyl déhydroalanine) dans 100 ml de méthanol contenant 0,54 g de méthalonate de sodium.

Par évaporation du solvant on obtient un solide blanc.

La recristallisation dans l'isopropanol fournit 2,25 g (87%) de sel sodique.

Point de fusion: 112 °C (polymérisation)

RMN ($D_2O$) δ (ppm): 7.0–6.5(4H,m) 5.7(1H,s) 5.4(1H,s) 4.5($H_2O$) 3.5(3H,s) 3.3(2H,s).

## Revendications

1. Composés énamides acylés caractérisés en ce que ce sont des composés répondant à la formule générale

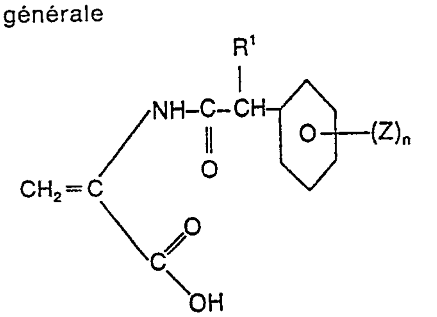

(I)

dans laquelle

– Z représente un groupement donneur d'électrons,

– n vaut 1, 2 ou 3,

– $R^1$ représente l'hydrogène, un halogène, un groupement aliphatique, substitué ou non, et comptant de 1 à 4 atomes de carbone ou un groupement aromatique comptant de 5 à 8 atomes de carbone,

ou des sels, complexes, dérivés alkylés ou dérivés acylés de ces composés.

2. Composés énamides acylés selon la revendication 1, caractérisés en ce que Z est un halogène ou un groupement sélectionné parmi les groupements $OR^2$, $SR^2$, $SeR^2$, $N=R^2$ et $NR^2R^3$ dans lesquels, l'oxygène, le soufre, le sélénium et l'azote peuvent faire partie d'un cycle, $R^2$ et $R^3$ peuvent être identiques ou différents et représentent l'hydrogène ou un groupement alkyle substitué ou non comptant de 1 à 6 atomes de carbone.

3. Composés énamides acylés selon la revendication 1 ou 2, caractérisés en ce que Z est un groupement $OR^2$ où $R^2$ représente l'hydrogène.

4. Composés énamides acylés selon l'une quelconque des revendications 1 à 3, caractérisés en ce qu'ils sont constitués de (méthoxyphénylacétyl) déhydroalanines.

5. Composés énamides acylés selon l'une quelconque des revendications 1 à 4 pour leur utilisation comme médicament.

6. Compositions pharmaceutiques caractérisées en ce qu'elles contiennent un composé énamide acylé selon l'une quelconque des revendications 1 à 4.

7. Compositions pharmaceutiques selon la revendication 6, caractérisées en ce qu'elles contiennent en outre une substance antinéoplasique.

8. Compositions pharmaceutiques selon la revendication 7, caractérisées en ce que la substance antinéoplasique est une moutarde à l'azote, un antibiotique ou un antipyrimidique.

9. Compositions pharmaceutiques selon la revendication 8, caractérisées en ce que la substance antinéoplasique est le cyclophosphamide ou la doxorubicine.

10. Compositions pharmaceutiques selon l'une quelconque des revendications 6 à 9 pour le traitement et la prévention des cancers.

11. Utilisation des composés énamides acylés selon l'une quelconque des revendications 1 à 4 pour l'obtention d'un médicament destiné au traitement et la prévention des cancers.

12. Procédé pour la préparation des composés énamides acylés selon l'une quelconque des revendications 1 à 4, caractérisé en ce que l'on fait réagir un amide de formule

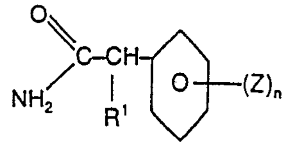

avec l'acide pyruvique.

## Claims

1. Acylated enamide compounds, characterised in that they are compounds corresponding to the general formula

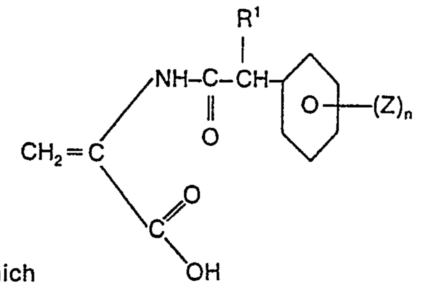

(I)

in which

Z represents an electron donor group,

n is 1, 2 or 3,

$R^1$ represents hydrogen, a halogen, a substituted or unsubstituted aliphatic group having 1 to 4 carbon atoms or an aromatic group having 1 to 8 carbon atoms,

or salts, complexes, alkylated derivatives or acylated derivatives of these compounds.

2. Acylated enamide compounds according to claim 1, characterised in that Z is a halogen or a group chosen from amongst the $OR^2$, $SR^2$, $SeR^2$, $N{=}R^2$ and $NR^2R^3$, in which the oxygen, sulphur, selenium and nitrogen can form part of a ring, $R^2$ and $R^3$ can be identical or different and represent hydrogen or a substituted or unsubstituted alkyl group having 1 to 6 carbon atoms.

3. Acylated enamide compounds according to claim 1 or 2, characterised in that Z is an $OR^2$ group, where $R^2$ represents hydrogen.

4. Acylated enamide compounds according to any one of claims 1 to 3, characterised in that they consist of (methoxyphenylacetyl)-dehydroalanines.

5. Acylated enamide compounds according to any one of claims 1 to 4, used as a medicament.

6. Pharmaceutical compositions characterised in that they contain an acylated enamide compound according to any one of claims 1 to 4.

7. Pharmaceutical compositions according to claim 6, characterised in that they moreover contain an antineoplastic substance.

8. Pharmaceutical compositions according to claim 7, characterised in that the antineoplastic substance is a nitrogen mustard, an antibiotic or anti-pyrimidine compound.

9. Pharmaceutical compositions according to claim 8, characterised in that the antineoplastic substance is cyclophosphamide or doxorubicin.

10. Pharmaceutical compositions according to any one of claims 6 to 9, used in the treatment and prevention of cancers.

11. Use of the acylated enamide compounds according to any one of claims 1 to 4 for the preparation of a medicament intended for the treatment and prevention of cancers.

12. Process for the preparation of acylated enamide compounds according to any one of claims 1 to 4, characterised in that an amide of the formula

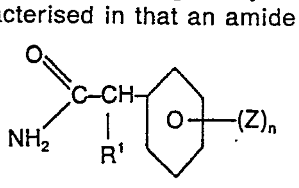

is reacted with pyruvic acid.

## Patentansprüche

1. Acylierte Enamidverbindungen, dadurch gekennzeichnet, daß sie Verbindungen sind entsprechend der allgemeinen Formel

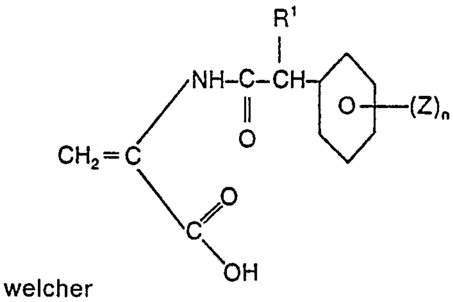

(I)

in welcher

– Z eine Elektronendonatorgruppe darstellt,

– n 1, 2 oder 3 ist,

– $R^1$ Wasserstoff, ein Halogen, eine aliphatische Gruppe, substituiert oder nicht, aufweist und von 1 bis 4 Kohlenstoffatome zählt oder eine aromatische Gruppe aufweist, die von 5 bis 8 Kohlenstoffatome zählt,

oder Salze, Komplexe, alkylierte Derivate oder acylierte Derivate dieser Verbindungen.

2. Acylierte Enamidverbindungen nach Anspruch 1, dadurch gekennzeichnet, daß Z ein Halogen oder eine Gruppe ist, die unter den Gruppen $OR^2$, $SR^2$, $SeR^2$, $N{=}R^2$ und $NR^2R^3$ ausgewählt ist, bei welchen der Sauerstoff, der Schwefel, das Selen und der Stickstoff zu einer Ringverbindung gehören können und $R^2$ und $R^3$ identisch oder unterschiedlich sein können und Wasserstoff oder eine Alkylgruppe aufweisen, substituiert oder nicht, die von 1 bis 6 Kohlenstoffatome zählt.

3. Acylierte Enamidverbindungen nach Anspruch 1 oder 2, dadurch gekennzeichnet, daß Z eine Gruppe $OR^2$ ist, wo $R^2$ Wasserstoff aufweist.

4. Acylierte Enamidverbindungen nach einem der Ansprüche 1 bis 3, dadurch gekennzeichnet, daß sie aus Dehydroalaninen (Methoxyphenylacetyl) bestehen.

5. Acylierte Enamidverbindungen nach einem der Ansprüche 1 bis 4 zur Verwendung als Medikament.

6. Pharmazeutische Zusammensetzungen, dadurch gekennzeichnet, daß sie eine acylierte Enamidverbindung nach einem der Ansprüche 1 bis 4 enthalten.

7. Pharmazeutische Zusammensetzungen nach Anspruch 6, dadurch gekennzeichnet, daß sie ferner eine Antineoplasiensubstanz enthalten.

8. Pharmazeutische Zusammensetzungen nach Anspruch 7, dadurch gekennzeichnet, daß die Antineoplasiensubstanz ein Stickstoffsenf, ein Antibiotikum oder ein antipyrimidisches Mittel ist.

9. Pharmazeutische Zusammensetzungen nach Anspruch 8, dadurch gekennzeichnet, daß die Antineoplasiensubstanz die Cyclophosphamidverbindung oder die Doxorubicinverbindung ist.

10. Pharmazeutische Zusammensetzungen nach einem der Ansprüche 6 bis 9 für die Behandlung und die Verhütung von Krebs.

11. Verwendung von acylierten Enamidverbindungen nach einem der Ansprüche 1 bis 4, um ein

Medikament zu erhalten, welches für die Behandlung und Verhütung von Krebs bestimmt ist.

12. Verfahren zur Gewinnung von acylierten Enamidverbindungen nach einem der Ansprüche 1 bis 4, dadurch gekennzeichnet, daß man ein Amid der Formel

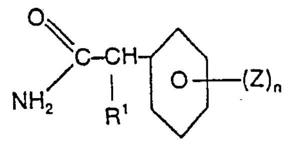

mit der Brenztraubensäure reagieren läßt.